# EUROPEAN PATENT APPLICATION

(11) **EP 4 374 864 A1**
(43) Date of publication of application: **29.05.2024**
(21) Application number: 22209149.8
(22) Date of filing: 23.11.2022
(51) Int. Cl.: A61K 36/185, A61P 25/00, B01D 11/00

(54) **METHOD FOR PREPARING THC-REDUCED CANNABIS PLANT EXTRACT**

(71) Applicant: Bioactive Botanicals Swiss AG, 8592 Uttwil (CH)
(72) Inventor: Kreuter, Emanuel, 8880 Walenstadt (CH); Berger Bueter, Karin, 8592 Uttwil (CH); Bueter, Bernhard, 8592 Uttwil (CH); Spiess, Stefan, 83646 Bad Toelz (DE)
(74) Representative: Kasche & Partner

(57) **Abstract**

The present invention is directed to a method for preparing a THC-reduced cannabis plant extract by mixing cannabis plant material with an aqueous organic solvent composition, optionally adjustting the pH of the aqueous plant extract to a pH of about 8 to about 11 by adding a base or base composition if the pH of the aqueous organic solvent composition is lower than about pH 8,-and separating solids from the basic aqueous plant extract, wherein for separating the solids in step (iii) the pH of the aqueous plant extract is about 8 to about 11; the organic solvent in the aqueous plant extract is selected from the group consisting of alcohols, aldehydes and ketones; and the organic solvent content in the aqueous plant extract is at least 10 % w/w. Furthermore, the present invention relates to a cannabis plant extract obtained or obtainable by said method, its use in the treatment of a disease, and food or feed, food or feed additive comprising said cannabis plant extract.

## Description

The present invention is directed to a method for preparing a THC-reduced cannabis plant extract by mixing cannabis plant material with an aqueous organic solvent composition, optionally adjusting the pH of the aqueous plant extract to a pH of about 8 to about 11 by adding a base or base composition if the pH of the aqueous organic solvent composition is lower than about pH 8, and separating solids from the basic aqueous plant extract, wherein for separating the solids in step (iii) the pH of the aqueous plant extract is about 8 to about 11; the organic solvent in the aqueous plant extract is selected from the group consisting of alcohols, aldehydes and ketones; and the organic solvent content in the aqueous plant extract is at least 15 % w/w. Furthermore, the present invention relates to a cannabis plant extract obtained or obtainable by said method, its use in the treatment of a disease, and food or feed, food or feed additive comprising said cannabis plant extract.

Methods for preparing plant extracts typically require a solvent system and extract conditions that preferably dissolve and extract the relevant ingredients, often medicinal components, and leave the undesired plant components undissolved and part of the sediment.

A problem with many plant extraction procedures and solvent systems is that, depending on the extracted plant material and the polarity of the hydrophilic and hydrophobic plant extract ingredients, the extract does not reflect the original natural active ingredient spectrum of the plant. Consequently, there often is no "drug equivalency" between the drug plant and its extract. For example, when using lipophilic extraction solvents hydrophilic ingredients are left almost unextracted, and moreover, lipophilic structural and metabolic ingredients of plant material tend to reduce the extracts dissolution ability aqueous media. As an additional result, many plant extracts encompass lipophilic contaminants such as herbicides, pesticides and/or undesired physiologically active plant ingredients, e.g. carcinogens. For examples, in some cannabis-based medicaments the psychotropic ingredient tetrahydrocannabinol (THC) is undesired.

Next to its cannabinoids *Cannabis sativa L.* encompasses a whole spectrum of secondary ingredients in its blossoms and flower heads such as, e.g. flavonoids, flavonol glycosides, phenanthrenes, steroids, xanthones, xanthone glycosides, terpenes and biphenyls. These substances span a broad range of polarity form hydro- to lipophilic.

For cannabis it is known that the very lipophilic cannabinoids can be efficiently extracted by means of apolar, lipophilic solvents, however, essentially nearly without any further hydrophilic ingredients. Current methods for Cannabis extraction are mainly based on lipophilic extraction solvents such as supercritical CO₂, ethanol, typically 96 % v/v aqueous ethanol, hexane and other lipophilic solvents (see Lazarjani et al. Cannabis Res 3:32, 2021, https://doi.org/10.1186/s42238-021-00087-9).

US 10,941,102 B2 teaches a method for obtaining precipitated and isolated cannabinoid acids, in particular THCA and CBDA, from plant material by exposing said plant material to a strong hydroxide based aqueous alkaline solution, removing the solid plant material and subsequently acidifying the solute to precipitate cannabinoid acids in the absence of any organic solvent.

It is the objective of the present invention to provide a method for preparing cannabis plant extracts comprising a compound spectrum and content similar to the spectrum and content of the natural plant with the exception of THC (drug equivalency). Furthermore, the extract should encompass both hydrophilic and lipophilic secondary plant ingredients, the latter including substances featuring at least two phenolic hydroxyl groups, such as cannabidiolic acid (CBDA) and cannabidiol (CBD). And the psychotropic ingredient tetrahydrocannabinol (THC) should be reduced or fully depleted. The method should also be environmentally friendly, simple, and safe.

In a first aspect the present invention is directed to a method for preparing a THC-reduced cannabis plant extract, comprising the steps
(i) mixing cannabis plant material with an aqueous organic solvent composition to prepare an aqueous plant extract, the composition optionally having a pH of about 8 to about 11;
(ii) adjusting the pH of the aqueous plant extract from step (i) to a pH of about 8 to about 11, optionally by adding a base or base composition, if the pH of the aqueous organic solvent composition in step (i) is lower than pH 8; and
(iii) separating solids from the aqueous plant extract to prepare a liquid aqueous plant extract, wherein for separating the solids in step (iii)
   - the pH of the aqueous plant extract is about 8 to about 11;
   - the organic solvent in the aqueous plant extract is selected from the group consisting of alcohols, aldehydes and ketones; and
   - the organic solvent content in the aqueous plant extract is at least 10 % w/w, optionally at least 15 % w/w for alcohols, or at least 10 % w/w for aldehydes and/or ketones.

It was found that the extraction of cannabis plant material with an aqueous organic solvent composition pH adjusted to a pH of about 8 to about 11 before or after mixing with cannabis plant material will provide cannabis plant extracts encompassing both hydrophilic and lipophilic secondary plant ingredients, including substances with at least two phenolic hydroxyl groups, in particular cannabidiolic acid (CBDA) and cannabidiol (CBD). Furthermore, it could be demonstrated that the method of the invention significantly reduced the psychotropic ingredient tetrahydrocannabinol (THC). The method is simple, it requires merely mixing, pH control and solid separation. It is environmentally friendly and safe, in particular when environmentally friendly organic solvents and bases are used.

The term "pH of about 8 to about 11", as used herein, is meant to be interpreted according to generally accepted mathematical rules, i.e. to include 7.5 to about 11.4. The same applies to other number terms used throughout the specification, e.g. at least about 15 % w/w is meant to also include at least 14.5 to 14.9 % w/w. The terms % w/w, % wt/wt and % m/m (w/wt=weight, m= mass) are used interchangeably. And the term "organic solvent in the aqueous plant extract is selected from the group consisting of alcohols, aldehydes and ketones", as used herein, is meant to include mixtures of alcohols, aldehydes and/or ketones. The term does not exclude further minor contents of other organic solvents or dissolved solids other than alcohols, aldehydes and ketones, e.g. less than 5, 4, 3, 2, 1, 0.5, or less than 0.1 % w/w organic content.

In an optional embodiment, the method of the invention may further comprise the step of (iv) mixing the liquid aqueous plant extract of step (iii) with a lipophilic solvent composition resulting in a lipophilic solvent phase and separating the lipophilic solvent phase, thereby preparing a THC-depleted or THC-free liquid aqueous plant extract.

The further lipophilic solvent extraction step will even more reduce the remaining little THC, in many cases resulting in a completely or essentially THC-free liquid aqueous plant extract. The terms "THC-depleted", "THC-free" and "essentially THC-free", as used herein, are meant to define a composition comprising no or at most 0.01 % w/w THC (depleted), whereas essentially THC-free indicates an amount of THC of less than 0.1 % w/w THC (essentially free), and THC-free means non-detectable THC in the extract composition.

The term "THC-reduced cannabis plant extract", as used herein, indicates an extract, wherein the extract does not include the full THC content of the extracted plant material, optionally less than 20, 10, 5, 1, or 0,1 % w/w of the THC content in the extracted plant.

The material for use in the extraction method of the invention may be any plant parts of Cannabis that comprises cannabidiol (CBD) and/or CBD derivatives, e.g. CBDA. For example, the cannabis plant material for the claimed method can be selected from the group consisting of cannabis whole plant material, cannabis root material, cannabis stem material, cannabis flower head, and cannabis blossoms, optionally cannabis blossoms and/or cannabis flower heads. For example, the cannabis plant material may be derived from Cannabis variants selected from the group consisting of Cannabis sativa L., C. americana Pharm., C. chinensis, C. europaea, C. indica and Polygonum viridiflorum. Plant material from C. sativa is particularly useful for practicing the present invention.

The term "aqueous organic solvent composition", as used herein, is meant to indicate compositions comprising at least water and organic solvent(s), optionally comprising at least water and physiologically acceptable organic solvents, e.g. solvents that are not toxic if consumed in appropriately small amounts. Optionally, the organic solvents for use in the present invention may be selected from the group consisting of aqueous organic compositions comprising C₁-C₆ alcohols, optionally linear C₁-C₄ alcohols, optionally ethanol and methanol, aqueous organic compositions comprising C₂-C₅, optionally C₂-C₃ aldehydes, and aqueous organic compositions comprising C₃-C₄ ketones, optionally acetone. The water content in the aqueous organic compositions is optionally at least 20, 25, 30, 40, 50, 60, 70, 80 or 90 % w/w. For example, the organic content in the aqueous organic compositions can be selected from about 10 to 80, 20 to 55, 35 to 45 % w/w.

An excellent organic solvent for practicing the method of the present invention is ethanol, in particular because of its solvent properties but also for its safety, environmental safety and physiological acceptability in mammals. In one optional embodiment of the present invention the aqueous organic solvent composition is a water-ethanol composition with an ethanol content of 10 to 80, 20 to 60 or 35 to 50 % % w/w.

The base for adjusting the pH in the aqueous organic compositions to 8 to 11, either before or after mixing with the plant extract material may be any suitable base, in particular a biologically and/or physiologically acceptable base or a base composition comprising one or more bases, optionally selected from the group consisting of sodium, potassium, ammonium, calcium, and magnesium hydroxide or -carbonate, and ammonia.

In alternative embodiments for practicing the method of the invention the pH of the aqueous organic, optionally physiologically acceptable solvent composition in step (i) or (ii) is from about 8.5 to about 10.5, optionally about 9 to about 10.

After separating the solid fraction from the basic aqueous organic compositions, i.e. after step (iii) or (iv), the pH of the liquid aqueous plant extract may optionally be adjusted to about 5 to about 8, optionally about 5 to about 7 or about 6 by addition of an optionally physiologically acceptable acid or acid composition, optionally aqueous compositions comprising an acid selected from the group consisting of hydrochloric acid, citric acid, acetic acid, carbonic acid and ascorbic acid.

The pH adjustment may be used to make the extract more physiologically acceptable for further use, in particular for formulating medicinal plant extracts.

The term "separating solids from the aqueous plant extract to prepare a liquid aqueous plant extract" is meant to indicate all common separation methods suitable for separating solid from liquid compositions, e.g. but not limited to decanting, separation by suction of the liquid, centrifugation, filtering with/without vacuum, etc.

The liquid aqueous organic plant extract obtained by the method of the invention may be partially or fully evaporated, e.g. to a spissum extract or dry extract, optionally after addition of physiologically acceptable excipients.

The term "spissum extract", as used herein, is meant to indicate that thick extracts are thickly liquid or viscous when warm, but are no longer fluid at room temperature.

The term "dry extract", as used herein, is meant to indicate less than 10, 7 or 5 % w. water.

Excipients for plant extract and extract containing medicaments in general are the same as for other medicaments.

In one embodiment for step (iv) of the method of the invention the optionally physiologically acceptable lipophilic solvent composition (phase) comprises one or more unsaturated or saturated plant fats, oils or waxes, optionally selected from the group consisting of coconut fat, peanut fat and bee wax.

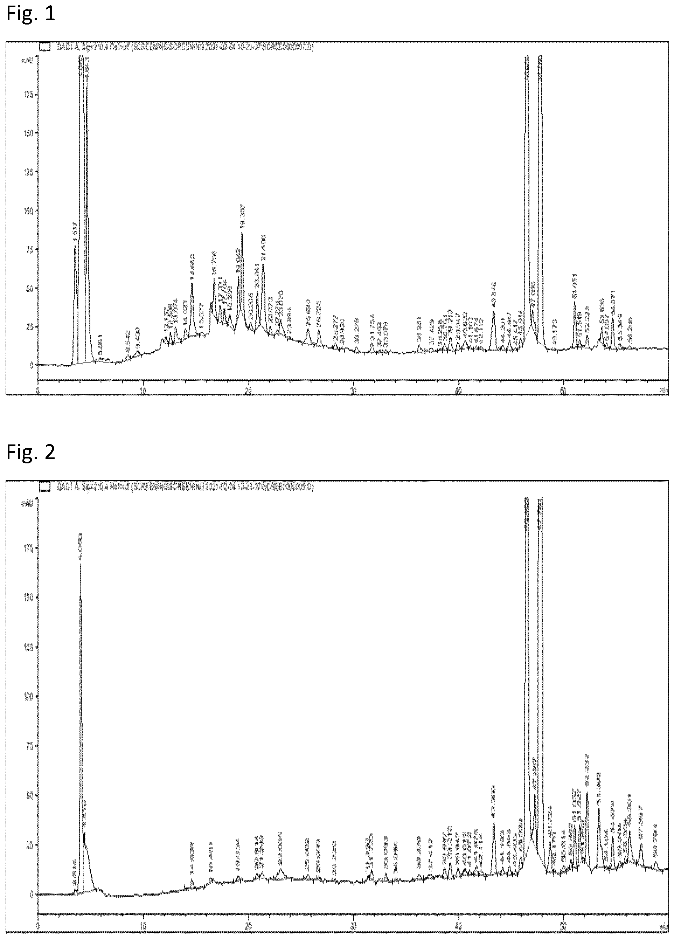

In a further aspect the present invention is directed to a cannabis plant extract, obtained or obtainable by a method of the present invention as described herein. It has been surprisingly found that the Cannabis extracts prepared according to the present invention encompass a broad (polarity) spectrum of secondary ingredients, optionally extracted from Cannabis blossoms and flower heads, such as, e.g. flavonoids, flavonol glycosides, phenanthrenes, steroids, xanthones, xanthone glycosides, terpenes and biphenyls, as illustrated in the differences of the HPLC chromatograms of examples 4 (figure 2, standard extract) and example 7 (figure 1, inventive extract). And these extracts are reduced in THC but high in cannabidiol and derivatives thereof. The cannabis plant extracts of the present invention feature new and very unique compositions. For example, the new extracts comprise extract components in much higher concentrations than conventional lipophilic extracts. Representative examples 1 to 3 and 5 to 8 show much higher cannabinoid contents when compared to non-alkalized conventional extracts. And HPLC results of the representative cannabis plant extracts of the present invention demonstrate a much bigger spectrum of substances in higher amounts.

Cannabis plant extracts obtainable by the method of the invention have use in the treatment of a disease, optionally a disease selected from the group consisting of epilepsia, neuralgia, spasms, cachexia, anxiety, convulsiveness, psychosis, inflammation, neurodermitis and arteriosclerosis.

In this regard, the extracts obtained by the method of the invention can be formulated into a pharmaceutical composition comprising the cannabis plant extract obtained.

The cannabis plant extract obtainable by the method of the invention can also be used to prepare a food or feed, food or feed additive comprising the obtained cannabis plant extract.

In one embodiment of the invention, the cannabis plant extract, pharmaceutical composition, food or feed, food or feed additive of the invention comprise lipophilic cannabis plant compounds characterized by having two or more phenolic hydroxyl moieties, optionally cannabidiolic acid (CBDA) and cannabidiol (CBD).

In the following the present invention will be illustrated by representative examples, figures and data, none of which is meant to limit the scope of the invention beyond the appended claims.

### Figures

**Fig. 1** depicts an HPLC chromatogram of the extract obtained according to the present invention from example 7 showing many more peaks of hydrophilic to medium polar substances when compared to the peaks resulting from comparison extract 4 also recorded at 220 nm with a diode array detector.
**Fig. 2** depicts an HPLC chromatogram of comparison extract 4 recorded at 220 nm with a diode array detector.

### Examples

### Example 1 (comparison) - Experiment 11, 35% m/m ethanol, not alkalized, fluid extract.

Step 1: 20 g *Cannabis sativa* blossoms, dried and cut (< 1mm), were mixed with 200 g solvent 35% m/m ethanol (Drug: solvent ratio (DSR) 1:10). Extraction was performed for 1.5 hours while permanently stirring at RT (25°C).

Step 2: The pH was determined at the beginning and at the end of the extraction.

Steps 3 and 4: The liquid extract was obtained by filtering via a folded filter.

Analytical data: 165 g liquid extract with 4.16 g dry substance was obtained. This corresponds to an extract yield of 5.04 g (absolute, relative to 200g extraction solvent). The pH at the beginning was 5.99 and at the end of the extraction 6.09. The liquid extract had a content of the cannabinoids CBDA and CBD (HPLC, DAB 2018) of 99.6 mg CBDA and 12.8 mg CBD (absolute, relative to 200 g extraction solvent). In addition, the total THC content (THC-A and THC) was determined after step 4 to be 0.20 mg (absolute, relative to 200 g extraction solvent).

### Example 2 (comparison) - Experiment 12, 40% m/m ethanol, not alkalized, fluid extract.

Step 1: 20 g *Cannabis sativa* blossoms, dried and cut (< 1mm), were mixed with 200 g solvent 40% m/m ethanol (DSR 1:10). Extraction was performed for 1.5 hours while permanently stirring at RT (25°C).

Step 2: The pH was determined at the beginning and at the end of the extraction.

Steps 3 and 4: The liquid extract was obtained by filtering via a folded filter.

Analytical data: 170 g liquid extract with 3.74 g dry substance was obtained. This corresponds to an extract yield of 4.40 g (absolute, relative to 200g extraction solvent). The pH at the beginning was 5.96 and at the end of the extraction 6.01. The liquid extract had a content of the cannabinoids CBDA and CBD (HPLC, DAB 2018) of 167.8 mg CBDA and 38.6 8 mg CBD (absolute, relative to 200 g extraction solvent).

### Example 3 (comparison) - Experiment 13, 45 % m/m ethanol, not alkalized, fluid extract.

Step 1: 20 g *Cannabis sativa* blossoms, dried and cut (< 1mm), were mixed with 201 g solvent 45% m/m ethanol (DSR 1:10). Extraction was performed for 1.5 hours while permanently stirring at RT (25°C).

Step 2: The pH was determined at the beginning and at the end of the extraction.

Steps 3 and 4: The liquid extract was obtained by filtering via a folded filter.

Analytical data: 175 g liquid extract with 3.78 g dry substance was obtained. This corresponds to an extract yield of 4.32 g (absolute, relative to 200 g extraction solvent). The pH at the beginning was 5.96 and at the end of the extraction 6.01. The liquid extract had a content of the cannabinoids CBDA and CBD (HPLC, DAB 2018) of 221.2 mg CBDA and 69 mg CBD (absolute, relative to 200 g extraction solvent).

### Example 4 (standard comparison) - Experiment 14, 94 % m/m ethanol, not alkalized, fluid extract.

Step 1: 20 g *Cannabis sativa* blossoms, dried and cut (< 1mm), were mixed with 201 g solvent 94 % m/m ethanol (DSR 1:10). Extraction was performed for 1.5 hours while permanently stirring at RT (25°C).

Step 2: The pH was determined at the beginning and at the end of the extraction.

Steps 3 and 4: The liquid extract was obtained by filtering via a folded filter.

Analytical data: 183 g liquid extract with 1.6 g dry substance was obtained. This corresponds to an extract yield of 1.75 g (absolute, relative to 200 g extraction solvent). The pH at the beginning was 6.34 and at the end of the extraction 6.26. The liquid extract had a content of the cannabinoids CBDA and CBD (HPLC, DAB 2018) of 289.6 mg CBDA and 109.4 CBD (absolute, relative to 200 g extraction solvent). In addition, the total THC content (THC-A and THC) was determined to be 8.0 mg for 200 g extraction solvent).

### Example 5 (extract of the invention) Experiment 3, 35% m/m ethanol, alkalized, fluid extract

Step 1: 20 g *Cannabis sativa* blossoms, dried and cut (< 1mm), were mixed with 200 g solvent 35 % m/m ethanol (DSR 1:1).

Step 2: The pH was adjusted with 33.3 % m/m NaOH solution to 8.5 to 10, readjusted every 30 min, and extracted for 1.5 hours while permanently stirring at RT (25°C).

Steps 3 and 4: The liquid extract was then obtained by filtering via a folded filter.

Step 5: The obtained filtrate was subsequently heated to 40 °C while permanently stirring and mixed with 2 g liquified coconut fat. This mixture was stirred for 20 minutes and then cooled in a deep freezer at -18 °C overnight.

Step 6: The solidified lipid layer was separated and the liquid extract obtained.

Analytical data: 132 g liquid extract with 4.4 g dry substance was obtained. This corresponds to an extract yield of 6.34 g (absolute, relative to 200 g extraction solvent and after subtracting the introduced Na content from the NaOH). The pH at the beginning before alkalizing was 6.1, directly after alkalization pH 9.3, and at the end of the extraction at pH 9.3. The amount NaOH solution needed was 2.01 g. After step 4 the liquid extract had a content of the cannabinoids CBDA and CBD (HPLC, DAB 2018) of 242.6 mg CBDA and 14 mg CBD, and after step 6 the liquid extract had 249.4 mg CBDA and 7.4 mg CBD (absolute, relative to 200 g extraction solvent). In addition, the total THC content (THC-A and THC) was determined after step 4 as being non-detectable (n.d.).

### Example 6 (extract of the invention) - Experiment 4, 40% m/m ethanol, alkalized, fluid extract

Step 1: 20 g *Cannabis sativa* blossoms, dried and cut (< 1mm), were mixed with 200 g solvent 40 % m/m ethanol (DSR 1:10).

Step 2: The pH was adjusted with 33.3 % m/m NaOH solution to 8.5 to 10, readjusted every 30 min, and extracted for 1.5 hours while permanently stirring at RT (25°C).

Steps 3 and 4: The liquid extract was then obtained by filtering via a folded filter.

Analytical data: 150 g liquid extract with 4.26 g dry substance was obtained. This corresponds to an extract yield of 5.29 g (absolute, relative to 200 g extraction solvent and after subtracting the introduced Na content from the NaOH. The pH at the beginning before alkalizing was 6.0, directly after alkalization pH 9.3, and at the end of the extraction at pH 9.35. The amount NaOH solution needed was 2.04 g. After step 4 the liquid extract had a content of the cannabinoids CBDA and CBD (HPLC, DAB 2018) of 294.4 mg CBDA and 32.6 mg CBD, (absolute, relative to 200 g extraction solvent).

### Example 7 (Extract of the invention) - Experiment 5, 45% m/m ethanol, alkalised, fluid extract

Step 1: 20 g *Cannabis sativa* blossoms, dried and cut (< 1mm), were mixed with 200 g solvent 45 % m/m ethanol (DSR 1:10).

Step 2: The pH was adjusted with 33.3 % m/m NaOH solution to 8.5 to 10, readjusted every 30 min, and extracted for 1.5 hours while permanently stirring at RT (25°C).

Steps 3 and 4: The liquid extract was then obtained by filtering via a folded filter.

Analytical data: 161 g liquid extract with 4.24 g dry substance was obtained. This corresponds to an extract yield of 4.88 g (absolute, relative to 200 g extraction solvent and after subtracting the introduced Na content from the NaOH. The pH at the beginning before alkalizing was 5.94, directly after alkalization pH 9.5, and at the end of the extraction at pH 9.55. The amount NaOH solution needed was 2.04 g. After step 4 the liquid extract had a content of the cannabinoids CBDA and CBD (HPLC, DAB 2018) of 442.2 mg CBDA and 78.8 mg CBD, (absolute, relative to 200 g extraction solvent).

### Example 8 (Extract of the invention) - Experiment BBS NP E 001, 45% m/m ethanol, alkalisiert, dry extract

200.6 g *Cannabis sativa* blossoms, dried and cut (< 2 mm), were mixed with 1750 g solvent 45 % m/m ethanol (DSR 1:9). The pH was adjusted with 10 % m/m NaOH solution to 8.5 to 10, readjusted every 30 min, and extracted for 1.5 hours while permanently stirring at 45-55 °C (45°C). Subsequently, the liquid extract was filtered via a Büchner funnel. 1567 g liquid extract with a dry substance content of 55.4 g was obtained. The pH at the beginning before alkalizing was 5.94, directly after alkalization pH 9.5, and at the end of the extraction at pH 10.0. This was achieved by repeated addition of NaOH solution. The amount NaOH solution needed was 124 g.

The so obtained filtrate was subsequently heated to 40 °C while permanently stirring and mixed with 20.79 g liquified coconut fat. This mixture was stirred for 20 Minutes and then cooled overnight in a deep freezer at -18 °C, and the lipid layer was removed after solidification. The so obtained thick extract was mixed with maltodextrin (Glucidex 12^{®}) and subsequently dried in a vacuum dry shelf at 50 °C and 10 mbar.

62.64 g dry extract were obtained with a drying loss of 1.24 %. This corresponds to an extract yield of 37 g (relative to the dry extract and after subtracting the introduced excipients maltodextrin, the introduced Na and CI content and the drying loss). The dry extract had a content of the cannabinoids CBDA and CBD (HPLC, DAB 2018) of 7704.7 mg CBDA and 3069.4 mg CBD, (relative to dry extract yield of 62.64 g.

### Results

Figures 1 and 2 show HPLC chromatograms for the extract of the invention according to Example 7 and the standard comparison extract from Example 4 (current state of the art extraction method). The HPLC method detects and quantifies ingredients of Cannabis over a broad range of polarity by separating hydrophilic, medium polar and apolar substances. More hydrophilic substances elute at the beginning, the lipophilic substances come last.

From the chromatograms it is clear that the extract of the invention features a much higher number of hydrophilic to medium polar substances than the comparison extract. The huge amount of peaks in the first 30min of the HPLC chromatogram of the inventive extract is nearly completely absent in the standard extract HPLC run. Both extracts were injected in identical amounts and such direct comparable.

Examples 5 to 8 demonstrate that the method of the invention produces liquid and dry extracts from Cannabis plant material, e.g. blossoms, with polar, in particular aqueous organic solvents that comprise the desired cannabinoids CBDA and CBD in quantities which can typically be extracted only with very lipophilic extraction solvents (see standard comparison extract of example 4). However, under these highly lipophilic conditions of the state of the art hydrophilic substances will not or insufficiently dissolve, and instead lipophilic substances and contaminants such as, e.g. polychlorinated carbohydrates and polyaromatics will be extracted from the environment and plant material. The method of the invention does not have this drawback. Specifically in experiments 5 and 6 an additional step for removing undesired lipophilic ingredients is introduced. According to Example 5 the sum of CBDA and CBD after steps 5 und 6 of the inventive method is comparable to the CBDA and CBD content after step 4: Step 4: 256.6mg and Step 6: 256.8mg.

The comparison examples 1 to 3 demonstrate that extraction solvents with a content of 35-45% m/m ethanol are not capable of extracting the desired ingredients CBDA and CBD even similar to the corresponding Examples of the invention 5 to 7. Example 7 according to the present invention further demonstrates that the inventive extraction method results in even higher yields of the desired cannabinoids CBDA and CBD than when extracting with the lipophilic extraction method of Example 4. Moreover, inventive examples 5 to 7 demonstrate relative to comparison examples 1 to 3 even higher extraction yields. Last but not least, the method of the invention as illustrated in Example 5 demonstrates that the THC content is not increased relative to the comparison extract of Example 1 by alkalizing the pH to 8 to 11, and that the THC content is negligible when compared to the standard comparison extract of Example 4, which is the method commonly used in the state of the art.

## Claims

1. Method for preparing a THC-reduced cannabis plant extract, comprising the steps
(i) mixing cannabis plant material with an aqueous organic solvent composition to prepare an aqueous plant extract;
(ii) adjusting the pH of the aqueous plant extract from step (i) to a pH of about 8 to about 11, optionally by adding a base or base composition, if the pH of the aqueous organic solvent composition in step (i) is lower than about pH 8; and
(iii) separating solids from the aqueous plant extract to prepare a liquid aqueous plant extract,
wherein for separating the solids in step (iii) the pH of the aqueous plant extract is about 8 to about 11; the organic solvent in the aqueous plant extract is selected from the group consisting of alcohols, aldehydes and ketones; and the organic solvent content in the aqueous plant extract is at least about 10 % w/w, optionally at least about 15 % w/w for alcohols, or at least about 10 % w/w for aldehydes and/or ketones.

2. The method of claim 1, further comprising the step of
(iv) mixing the liquid aqueous plant extract of step (iii) with a lipophilic solvent composition resulting in a lipophilic solvent phase and separating the lipophilic solvent phase, thereby preparing a THC-depleted or THC-free liquid aqueous plant extract.

3. The method of claim 1 or 2, wherein the cannabis plant material is selected from the group consisting of cannabis whole plant material, cannabis root material, cannabis stem material, cannabis flower head, and cannabis blossoms, optionally cannabis blossoms and/or cannabis flower heads.

4. The method of any of claims 1 to 3, wherein the aqueous organic solvent composition is selected from the group consisting of aqueous organic compositions comprising C₁-C₆ alcohols, optionally linear C₁-C₄ alcohols, optionally ethanol and propanol, aqueous organic compositions comprising C₂-C₅, optionally C₂-C₃ aldehydes, and aqueous organic compositions comprising C₃-C₄ ketones, optionally acetone.

5. The method of claim 4, wherein the aqueous organic solvent composition is a water-ethanol composition with an ethanol content of 10 to 80, 20 to 60 or 35 to 50 % w/w.

6. The method of any of claims 1 to 5, wherein the base is or the base composition comprises one or more bases selected from the group consisting of sodium, potassium, calcium, and magnesium, ammonium hydroxide or -carbonate, and ammonia.

7. The method of any of claims 1 to 6, wherein the pH of the aqueous organic solvent composition in step (i) or (ii) is from about 8.5 to about 10.5, optionally about 9 to about 10.

8. The method of any of claims 1 to 7, wherein after step (iii) or (iv) the pH of the liquid aqueous plant extract is adjusted to about 5 to about 8, optionally about 5 to about 7 or about 6 by addition of a physiologically acceptable acid or acid composition, optionally aqueous compositions comprising an acid selected from the group consisting of hydrochloric acid, citric acid, acetic acid, carbonic acid and ascorbic acid.

9. The method of any of claims 1 to 8, wherein the liquid aqueous organic plant extract is evaporated to a spissum extract or dry extract, optionally after addition of physiologically acceptable excipients.

10. The method of any of claims 2 to 9, wherein the physiologically acceptable lipophilic solvent composition phase comprises one or more unsaturated or saturated plant fats or waxes, optionally selected from the group consisting of coconut fat, peanut fat, and bee wax.

11. A cannabis plant extract, obtained or obtainable by a method according to any of claims 1 to 10.

12. The cannabis plant extract according to claim 11 for use in the treatment of a disease, optionally a disease selected from the group consisting of epilepsia, neuralgia, spasms, cachexia, anxiety, convulsiveness, psychosis, inflammation, neurodermitis and arteriosclerosis.

13. A pharmaceutical composition comprising a cannabis plant extract according to claim 11.

14. A food or feed, food or feed additive comprising a cannabis plant extract according to claim 11.

15. The cannabis plant extract, pharmaceutical composition, food or feed, food or feed additive according to any of claims 11 to 14 comprising lipophilic cannabis plant compounds **characterized by** having two or more phenolic hydroxyl and/or one or more acid moieties, optionally cannabidiolic acid (CBDA) and cannabidiol (CBD).
